# EUROPEAN PATENT APPLICATION

(11) **EP 4 772 035 A1**
(43) Date of publication of application: **08.07.2026**
(21) Application number: 24860136.1
(22) Date of filing: 03.07.2024
(51) Int. Cl.: A24F 23/02, A24B 13/00, A24B 15/16, A24B 15/24, A24B 15/30, A24B 15/32, A24B 15/42, A24B 15/28, A24B 15/38, B65B 29/02

(54) **NICOTINE POUCH AND PACKAGING METHOD THEREFOR**

(30) Priority: 31.08.2023 KR 20230115723
(71) Applicant: KT&G Corporation, Daedeok-gu Daejeon 34337 (KR)
(72) Inventor: JEOUNG, Eun Mi, Daejeon 34128 (KR); KI, Sung Jong, Daejeon 34128 (KR); CHA, Sung Je, Daejeon 34128 (KR); SHIN, Jun Won, Daejeon 34128 (KR)
(74) Representative: Ter Meer Steinmeister & Partner
(86) International application number: PCT/KR2024/009345
(87) International publication number: WO 2025/048212

(57) **Abstract**

The present invention relates to a nicotine pouch comprising a nicotine pouch filler and a packaging material, wherein the volume filling rate of the nicotine pouch filler is 60% to 75% on the basis of the internal volume of the nicotine pouch.

## Description

### TECHNICAL FIELD

One or more embodiments relate to a nicotine pouch and a method of packaging the same.

### BACKGROUND ART

Nicotine pouches are one type of smoking article and are used in the mouth of a user to provide nicotine satisfaction to the user. For example, nicotine pouches may be used by a user placing the pouch between the upper or lower gums and lips and holding it there for a limited period of time.

A nicotine pouch includes a filler packaged in a packaging material. The packaging material holds a filler in place and allows saliva to pass through the filler, and a flavor and nicotine released from the filler diffuse into the user's mouth.

Therefore, there is a need for a nicotine pouch that may release most or all of the nicotine in an appropriate amount from a nicotine pouch filler and is not uncomfortable when used in a mouth.

### DISCLOSURE OF THE INVENTION

### TECHNICAL GOALS

Embodiments provide a nicotine pouch having excellent nicotine elution rate and feeling of use.

However, goals to be achieved are not limited to those described above, and other goals not mentioned above are clearly understood by one of ordinary skill in the art from the following description.

### TECHNICAL SOLUTIONS

According to an aspect, there is provided a nicotine pouch including a nicotine pouch filler, and a packaging material, wherein a volume filling rate of the nicotine pouch filler is 60% to 75% of an internal volume of the nicotine pouch.

According to another aspect, there is provided a method of packaging a nicotine pouch, the method including wrapping a nicotine pouch filler with a packaging material, and sealing the packaging material, wherein the nicotine pouch filler has a volume filling rate of 60% to 75% of an internal volume of the nicotine pouch.

### EFFECTS OF THE INVENTION

When a nicotine pouch and a packaging method are used, nicotine may be effectively eluted from a nicotine pouch filler and comfortable feeling of use may be provided.

It should be understood that the effects of the present disclosure are not limited to the above-described effects, but are construed as including all effects that may be inferred from the configurations and features described in the following description or claims of the present disclosure.

### BRIEF DESCRIPTION OF THE DRAWINGS

These and/or other aspects, features, and advantages of the invention will become apparent and more readily appreciated from the following description of embodiments, taken in conjunction with the accompanying drawings of which:
FIG. 1 is a side view of a nicotine pouch according to an embodiment; and
FIG.2 a diagram for a result of a nicotine elution rate of a nicotine pouch of an embodiment.

### BEST MODE FOR CARRYING OUT THE INVENTION

Hereinafter, embodiments will be described in detail with reference to the accompanying drawings. However, various alterations and modifications may be made to the embodiments and thus, the scope of the disclosure is not limited or restricted to the embodiments. The equivalents should be understood to include all changes, equivalents, and replacements within the idea and the technical scope of the disclosure.

The terminology used herein is for the purpose of describing particular embodiments only and is not to be limiting of the embodiments. The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless disclosed to the contrary, the description of any one embodiment may be applied to other embodiments, and the specific description of the repeated configuration will be omitted.

It will be understood that when a certain part "includes" a certain component, the part does not exclude another component but may further include another component.

According to an embodiment, there is provided a nicotine pouch including a nicotine pouch filler, and a packaging material, wherein a volume filling rate of the nicotine pouch filler is 60% to 75% of an internal volume of the nicotine pouch.

The nicotine pouch filler may be packaged inside the packaging material, and an appropriate amount of the nicotine pouch filler may be filled to improve performance of the nicotine pouch and feeling of use. Desirably, the volume filling rate of the nicotine pouch filler may be 65% to 70% of the internal volume of the nicotine pouch.

When the volume filling rate of the nicotine pouch filler is less than the numerical range described above, smoking satisfaction may not be satisfied and it may slip easily in a mouth due to insufficient nicotine. On the other hand, when the volume filling rate of the nicotine pouch filler exceeds the numerical range described above, an excessive amount of nicotine pouch filler may be included to hinder nicotine elution, and a foreign body sensation may be felt due to hardness when used.

In the nicotine pouch according to an embodiment, the nicotine pouch may have a thickness of 2.8 mm to 4.5 mm, desirably 3 mm to 3.5 mm. When the thickness of the nicotine pouch is less than the numerical range described above, the nicotine pouch is not likely to be fixed between the upper gums and the oral cavity, and due to excessive flexibility, it may easily move, such as slipping during conversation or drinking, and a user may feel foreign body sensation. On the other hand, when the thickness of the nicotine pouch exceeds the numerical range described above, the foreign body sensation may be felt during use due to excessive hardness of the nicotine pouch when used in the mouth.

The internal volume of the nicotine pouch may vary depending on a size of the nicotine pouch.

The nicotine pouch may be classified into original, slim, super slim, and mini forms according to sizes and weights thereof. The original form has the most common size in snus products and is mainly used in North America. The slim form is the most common size in nicotine pouch products and is mainly used in Europe. The super slim form is a product for female consumers and has a smaller size than the slim form. The mini form is a small nicotine pouch and is mainly used in Europe.

In the nicotine pouch according to an embodiment, the size of the nicotine pouch may have a vertical length of 11 mm to 17 mm and a horizontal length of 25 mm to 32 mm. For example, the nicotine pouch may have a vertical length of 12 mm to 14 mm and a horizontal length of 28 mm to 30 mm.

In the nicotine pouch according to an embodiment, the nicotine pouch has a weight of 0.3 g to 0.6 g. The weight of the nicotine pouch may be a result obtained by adding weights of the nicotine pouch filler and the packaging material.

The size and weight of the nicotine pouch must meet the ranges described above to provide a nicotine pouch that is short in length, thin, and has a usage time of 20 to 40 minutes.

In the nicotine pouch according to an embodiment, the internal volume of the nicotine pouch may be 0.8 cm3 to 1.2 cm³. In an example, the internal volume of the nicotine pouch may be 1 cm³ to 1.1 cm³.

The packaging material may be packaged in three-sided packaging or back packaging, and sealing of the packaging material may be performed by ultrasonic sealing or heat sealing. The packaging material may be formed with a longitudinal seal formed along a horizontal direction of the packaging material and a cross seal formed along a vertical direction of the packaging material, and the longitudinal seal and/or cross seal may be sealed by lap sealing or fin sealing.

In the nicotine pouch according to an embodiment, the packaging material is packaged by lap sealing, and the packaging material may be sealed with a width of 1 mm to 3 mm. The lap sealing is a packaging method that may be used in a packaging machine, and may be an advantageous method for improving leakage prevention against an annular support surrounding an opening in an annular type packaging method. When a sealing width is excessively wide beyond the range described above, a user may feel a foreign body sensation caused by a sealing portion and sharpness of a cutting surface.

In the nicotine pouch according to an embodiment, the nicotine pouch filler may include nicotine, a release control excipient including at least one selected from a group consisting of cellulose and sugar alcohol, a binder, a pH adjuster, and a flavoring agent.

In the nicotine pouch filler, the nicotine may include nicotine salt, and the nicotine salt may include, for example, at least one selected from a group consisting of nicotine salt benzoate, nicotine salt tartrate, nicotine salt malate, and nicotine salt salicylate.

A content of the nicotine may be determined according to a desired strength of the nicotine pouch. When the content of the nicotine is less than the range described above, it is difficult to give the smoker the satisfaction of smoking. On the other hand, when the content of the nicotine exceeds the range described above, nicotine stimulation may be excessive.

In the nicotine pouch filler, the release control excipient may adjust a nicotine elution rate.

Cellulose in the release control excipient may function as a carrier for nicotine, quickly release saliva and nicotine, and provide feeling of use. The cellulose may be one or more of microcrystalline cellulose (MCC) or methyl cellulose.

The content of cellulose may be 1 to 2 times the content of nicotine. The content of cellulose may be 5 to 20 wt% with respect to a weight of total solid contents of the nicotine pouch filler. When the content of the cellulose is less than the numerical range described above, the nicotine pouch filler may dissolve quickly. On the other hand, when the content of the cellulose exceeds the numerical range described above, the nicotine elution rate may be delayed due to a water-insoluble characteristic of cellulose.

The sugar alcohol in the release control excipient is highly soluble in saliva and loosely binds to a solvent than the cellulose. Accordingly, the sugar alcohol may control the nicotine release within the time of use, suppress a bitter taste, and provide a cooling effect when dissolved in the mouth. In addition, the sugar alcohol may promote nucleation and granule growth in a high-shear wet granulation process of the nicotine pouch filler.

The sugar alcohol may include at least one selected from a group consisting of erythritol, xylitol, mannitol, sorbitol, maltitol, and isomalt. However, the sugar alcohol is not limited to the examples listed above.

A content of the sugar alcohol may be 50 to 70 wt%, desirably, 60 to 70 wt% with respect to the weight of the total solid contents of the nicotine pouch filler. When the content of the sugar alcohol is below the numerical range described above, nuclei of particles of the nicotine pouch filler may not be sufficiently formed. Meanwhile, when the content of the sugar alcohol exceeds the numerical range described above, severe agglomeration or crushing of a powder may occur, making it difficult to prepare the nicotine pouch filler.

A type and a content of the binder may affect the formation of a size distribution of nicotine pouch filler particles.

In the nicotine pouch filler, the binder may include at least selected from a group consisting of Povidone K-25, hydroxypropyl cellulose (HPC), low-substituted hydroxypropyl cellulose (L-HPC), hydroxypropylmethyl cellulose (HPMC), and carboxymethylcellulose (CMC).

A content of the binder may be 10 wt% or less, desirably, 8 to 15 wt% with respect to the weight of the total solid contents of the nicotine pouch filler. When the type and the content of the binder are outside the range described above, it may be difficult to secure a required particle size and may have a negative effect on initial elution.

The binder may be added by a wet binder adding method or a dry binder adding method.

The pH adjuster may adjust the pH of the nicotine pouch filler to a pH with which nicotine may be absorbed such that nicotine may be converted to a free-based nicotine form that may be absorbed quickly when the nicotine pouch is inserted into the mouth.

The pH of the nicotine pouch filler may be 8.3 to 8.5. When the pH is 8.02 or more, nicotine may be converted into the free-based nicotine form, which may be quickly absorbed through the mucous membrane. Therefore, when the pH of the nicotine pouch filler satisfies the numerical range described above, the pH may be 8.02 or more when mixed with saliva (pH 6.5 to 7.7), and thus, nicotine may be easily absorbed and have a strong impact on consumers.

The pH adjuster may be, for example, at least one selected from a group consisting of sodium bicarbonate and sodium carbonate. A weight ratio of the sodium bicarbonate to sodium carbonate may be 7:3 to 6:4, desirably 6:4. When a higher proportion of sodium bicarbonate is added than the ratio described above (a ratio with a relatively lower proportion of sodium carbonate), the pH may be lowered and nicotine may not be sufficiently absorbed. On the other hand, when a lower proportion of sodium bicarbonate is added than the ratio described above (a ratio with a relatively higher proportion of sodium carbonate), the pH may become higher than the desired range and may not be suitable for elution in the mouth.

The flavoring agent may provide a taste to satisfy the smoker's preference and is not limited to a specific flavoring agent. For example, the flavoring agent may include at least one selected from a group consisting of menthol, licorice, sucrose, fructose syrup, isosweetener, cocoa, lavender, cinnamon, cardamom, celery, fenugreek, cascarilla, sandalwood, bergamot, geranium, honey essence, rose oil, vanilla, lemon oil, orange oil, mint oil, caraway, cognac, jasmine, chamomile, cinnamon, ylang ylang, sage, spearmint, peppermint, ginger, coriander, and coffee.

In the nicotine pouch according to an embodiment, the nicotine pouch filler may include 65% to 75% of particles with a size of 200 µm or more, and 0% to 35% of particles with a size of 100 µm or more and less than 200 µm. Desirably, the nicotine pouch filler may include 65% to 70% of particles with a size of 200 µm or more, and 20% to 30% of particles with a size of 100 µm or more and less than 200 µm. A remainder may be particles with a size of 100 µm or less. The nicotine pouch filler may not include fine particles with a size of 50 µm or less.

When the size of the nicotine pouch filler satisfies the numerical range described above, nicotine may be efficiently released. When the content of the particles with a small size is excessively great in the nicotine pouch filler, the nicotine may not be released in the initial stage. On the other hand, when the content of the particles with a great size is excessively great in the nicotine pouch filler, all of the nicotine may be released in the initial stage and the nicotine may not be provided to a user in the later stages of use.

In the nicotine pouch according to an embodiment, a density of the nicotine pouch filler may be 0.34 g/cm³ to 0.74 g/cm³, desirably 0.4 g/cm³ to 0.6 g/cm³, and more desirably 0.53 g/cm³ to 0.55 g/cm³.

In the nicotine pouch according to an embodiment, a content of the nicotine pouch filler may be 280 mg to 460 mg, desirably 300 mg to 400 mg.

In the nicotine pouch according to an embodiment, a filling density of the nicotine pouch may be 0.26 g/cc to 0.44 g/cc, desirably 0.28 g/cc to 0.39 g/cc. The filling density may be expressed as a content of the nicotine pouch filler/an inner volume of the nicotine pouch.

When the content and the filling density of the nicotine pouch filler are less than the numerical ranges described above, a usage time of the nicotine pouch may be reduced, and an initial amount of nicotine delivered may increase and persistence may be reduced due to elution imbalance. On the other hand, when the content and the filling density of the nicotine pouch filler exceed the numerical ranges described above, an excessive amount of nicotine pouch filler may be contained to hinder nicotine elution.

In the nicotine pouch according to an embodiment, the nicotine pouch filler may be provided by the following preparation method. A method of preparing the nicotine pouch filler may include step S1 of mixing at least one selected from a group consisting of nicotine, a release control excipient, a binder, a pH adjuster, and a flavoring agent, step S2 of spraying a solvent, and step S3 of performing granulation. In step S1, the release control excipient may include at least one selected from a group consisting of cellulose and sugar alcohol, in step S2, the solvent may include at least one selected from a group consisting of water and alcohol, in step S2, a content of the solvent may be 10 to 60 wt% of a content of the cellulose, and in step S2, an L/S ratio may be 8% to 15%.

Hereinafter, each step will be described in detail.

Step S1 is step of mixing at least one selected from a group consisting of nicotine, a release control excipient, a binder, a pH adjuster, and a flavoring agent. At this time, the components may be prepared in a powder form and may be uniformly mixed.

The nicotine may include nicotine salt, and the type of nicotine salt is the same as the examples described above.

The release control excipient may include at least one selected from a group consisting of cellulose and sugar alcohol. The sugar alcohol in the release control excipient may promote nucleation and granule growth in the granulation step.

The type of the binder is the same as the examples described above. A method of adding the binder may particularly affect the granulation step. The binder may be added by a wet binder adding method or a dry binder adding method, desirably by a dry binder adding method. When the binder is added by the dry binder adding method, it is easy to form particles having a desired size, and a uniform particle size distribution may be obtained, thereby facilitating production.

The types of the pH adjuster and the flavoring agent are the same as the examples described above.

Step S2 is step of spraying a solvent. At this time, the solvent may be sprayed onto and mixed with a powder mixture prepared in step S1.

At this time, the solvent may be at least one of water or alcohol, desirably alcohol.

A content of the solvent may be 10 to 60 wt%, desirably 40 to 50 wt% of the content of the cellulose. The amount of solvent to be added may be represented by an L/S ratio. The L/S ratio is represented as a total weight of the solvent/a total weight of the solid powder mixture, and the total weight of the solid powder mixture may be a total weight of at least one selected form a group consisting of the nicotine, the release control excipient, the binder, the pH adjuster, and the flavoring agent added in step S1. The L/S ratio may be 8% to 15%, desirably 10% to 12%. When the L/S ratio satisfies the numerical range described above, the size is uniform, fine particles with a size of less than 100 µm are generated only in small amounts or not at all, and particles with a porous structure may be formed, which is advantageous in nicotine release. When the L/S ratio is less than the numerical range described above, the nicotine may not be easily eluted. On the other hand, when the L/S ratio exceeds the numerical range described above, irregular particles are formed, the particle size range becomes small, and the nicotine may be eluted excessively quickly.

Step S3 is step of performing granulation. At this time, after stirring the powder mixture prepared in step S2 and a solvent for 5 to 30 minutes, the clumps of particles agglomerated during the stirring process may be loosened and separated into individual particles.

According to an embodiment, the packaging material may include a cellulose fiber, and a thermoplastic material fiber.

The packaging material may be safe for the human body, hold saliva well, have a certain strength to prevent tearing, and have sealing properties.

The cellulose fiber may increase airtightness and physical strength of the packaging material for the nicotine pouch and also improve feeling of use (smoothness).

In an example, the cellulose fiber may include at least one selected from a group consisting of cellulose, viscose, rayon, and pulp.

When the cellulose fiber is pulp, the pulp may be beaten. The "beating" herein may refer to putting a fiber into water and cutting it to make them finer through a mechanical action. Pulp may be in a state of being easier to hydrogen bond as a length and a structure of the fiber change through the beating, thereby giving a physical strength to a pouch material.

Meanwhile, by including the thermoplastic material fiber, the packaging material for the nicotine pouch may be sealed with heat.

In an example, the thermoplastic material fiber may include at least one selected from a group consisting of nylon, polylactic acid, polyethylene, polypropylene, polyethylene/polypropylene, polyethersulfone, polyethylene terephthalate, and low-melting-point polyethylene terephthalate (LMPET).

In addition, in the packaging material for the nicotine pouch according to an embodiment, the thermoplastic material fiber may be a dual component fiber including two selected from a group consisting of polypropylene, polyethylene, polyethylene terephthalate, and polylactic acid, and the thermoplastic material fiber may form a core-shell structure. A fiber with such a core-shell structure may have the advantage of excellent physical properties and excellent sealing properties at low temperatures.

The thermoplastic material fiber may include a staple fiber. The staple fiber is a fiber with a discontinuous length and may be cut from a continuous filament.

The material of the packaging material is not limited to the components described above and may further include a binder and the like.

The packaging material may be a nonwoven type or woven type material.

A method of preparing the packaging material may include a process of forming a web and a process of bonding the web.

The process of forming the web may be a dry-laid method, a wet-laid method, or a spinning method, and the dry-laid method may be a carding method or an air ray method. The wet-laid method may be a general paper preparing method or a preparing method obtained by partially modifying it. The spinning method may be a spun-bonded or melt-blown method.

Meanwhile, the process of bonding the web may include a mechanical bonding method, a chemical bonding method, or a thermal bonding method. The mechanical bonding method may include a needle-punching method or a spun-lace method. The chemical bonding method may include a polymer dispersion method or a polymer solution method. The thermal bonding method may include a calendering method, a hot air method, an ultrasonic method, or the like.

Desirably, the packaging material may be prepared by the spun-lace method and/or the wet-laid method. The spun-lace method may include forming a web in a form of a sheet with a predetermined thickness with crumpled fiber raw material, forming a material of a packaging material by spraying moisture such that fibers are tangled, and drying. The wet-laid method may include preparing a dispersion by dispersing a fiber raw material in water, forming a material of a packaging material by moving the dispersion to a paper machine, and dehydrating and drying. However, the spun-lace method and the wet-laid method are not limited to including the processes described above.

The packaging material may have a porosity to release the nicotine pouch filler filled inside the packaging material.

According to an embodiment, there is provided a method of packaging a nicotine pouch, the method including wrapping a nicotine pouch filler with a packaging material, and sealing the packaging material, wherein the nicotine pouch filler has a volume filling rate of 60% to 75% of an internal volume of the nicotine pouch.

In the step of sealing the packaging material, a thermal or ultrasonic fusion method may be used, and at this time, the packaging material for the nicotine pouch may be sealed by adjusting conditions such as a temperature according to the type of fiber forming the packaging material and/or the manufacturing conditions of the packaging material.

Hereinafter, the present disclosure will be described in more detail with reference to examples, however, the present disclosure is not limited to the examples below.

### 1. Preparation of nicotine pouch

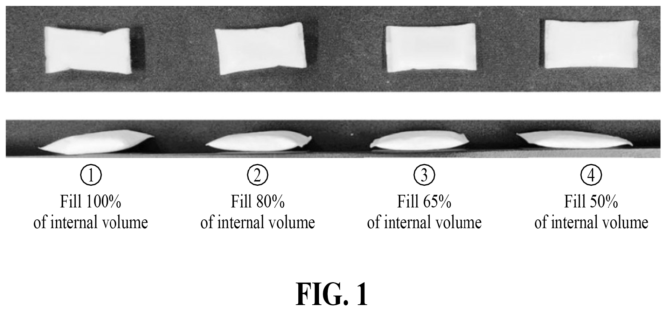

Nicotine pouches of Preparation Example 1 and Comparative Examples 1 to 3 were prepared by the following method (FIG. 1), and thicknesses of the nicotine pouches and nicotine pouch fillers added are as shown in Table 1.

### Preparation Example 1

A nicotine pouch in which a volume filling rate of a nicotine pouch filler is 65% of an internal volume of the nicotine pouch, and a filling density of the nicotine pouch filler is 0.35 g/cc was prepared. At this time, the nicotine pouch was prepared such that a longitudinal seal is sealed by lap sealing, and widths of the longitudinal seal and a cross seal were 2 mm. A finally sealed nicotine pouch had a size with a vertical length of 14 mm and a horizontal length of 28 mm. A density of the nicotine pouch filler included in the nicotine pouch was 0.54 g/cm³.

### Comparative Example 1

A nicotine pouch was prepared in the same manner as in Preparation Example 1 except that the volume filling rate of the nicotine pouch filler is 50% of the internal volume of the nicotine pouch, and the filling density of the nicotine pouch filler is 0.26 g/cc. The density of the nicotine pouch filler included in the nicotine pouch was 0.51 g/cm³.

### Comparative Example 2

A nicotine pouch was prepared in the same manner as in Preparation Example 1 except that the volume filling rate of the nicotine pouch filler is 80% of the internal volume of the nicotine pouch, and the filling density of the nicotine pouch filler is 0.45 g/cc. The density of the nicotine pouch filler included in the nicotine pouch was 0.56 g/cm³.

### Comparative Example 3

A nicotine pouch was prepared in the same manner as in Preparation Example 1 except that the volume filling rate of the nicotine pouch filler is 100% of the internal volume of the nicotine pouch, and the filling density of the nicotine pouch filler is 0.52 g/cc. The density of the nicotine pouch filler included in the nicotine pouch was 0.52 g/cm³.

**[Table 1]**

| Classification | Preparation Example 1 | Comparative Example 1 | Comparative Example 2 | Comparative Example 3 |
|---|---|---|---|---|
| Thickness of nicotine pouch (mm) | 3.2 | 2.74 | 4.6 | Maximum of 5 |
| Content of filler (mg) | 370 | 270 | 470 | 545 |

### 2. Experimental Example 1: Evaluation of nicotine elution rate according to volume filling rate

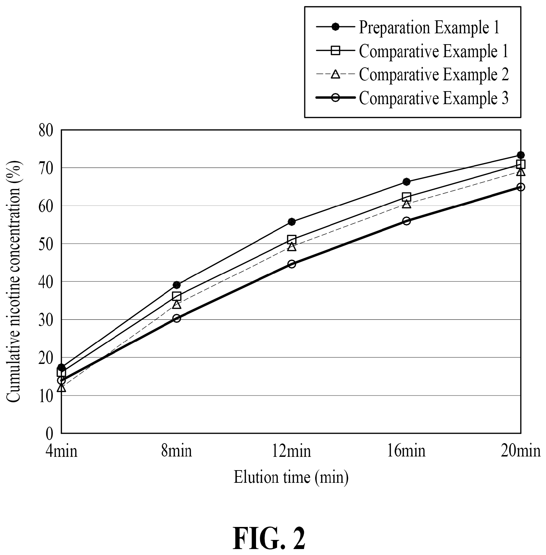

Nicotine elution rates of Preparation Example 1 and Comparative Examples 1 to 3 were investigated. A nicotine elution rate was evaluated by an in-house analysis method (modified USP4 method) based on Altria's nicotine elution analysis method. Artificial saliva was manufactured based on German standard DIN V Test Method 53160-1, elution intervals were 4 minutes, 8 minutes, 12 minutes, 16 minutes, 20 minutes, 30 minutes, 40 minutes, 50 minutes, and 60 minutes, and the analysis was performed by a nicotine quantitative method using UPLC-PDA using an internal standard solution. The results thereof are shown in Table 2 and FIG. 2.

**[Table 2]**

| Classification | Cumulative nicotine concentration (%) | |
|---|---|---|
| | After 4 minutes | After 20 minutes |
| Preparation Example 1 | 17.5 | 73.6 |
| Comparative Example 1 | 16.0 | 71.2 |
| Comparative Example 2 | 12.4 | 69.3 |
| Comparative Example 3 | 13.8 | 64.9 |

In Comparative Example 1, the initial elution of nicotine was fast, but the sustainability in the second half was poor, and in Comparative Examples 2 and 3, the nicotine elution rate was low overall.

Preparation Example 1, compared to Comparative Examples 1 to 3, had a highest cumulative nicotine content in the early and late stages, and an easiest volume filling rate for nicotine elution was confirmed.

### 3. Experimental Example 2: Evaluation of feeling of use according to volume filling rate

A sensory evaluation was conducted to evaluate the feeling of use of Preparation Example 1 and Comparative Examples 1 to 3. The sensory evaluation was conducted as five to six panel members used the nicotine pouches in the mouth. The results thereof are shown in Table 3.

**[Table 3]**

| Classification | Evaluation of feeling of use |
|---|---|
| Preparation Example 1 | Comfortable |
| Comparative Example 1 | Nicotine pouch is difficult to hold in mouth and slides or moves |
| Comparative Example 2 | Uncomfortable due to foreign body sensation of hardness during use |
| Comparative Example 3 | Uncomfortable due to foreign body sensation of hardness during use |

It was confirmed that Preparation Example 1, unlike Comparative Examples 1 to 3, provided comfortable feeling of use during use.

From the experimental examples above, it is predicted that, by using the nicotine pouch and the packaging method described in the claims, it is possible to provide the comfortable feeling of use with an excellent nicotine elution rate.

While the embodiments are described with reference to drawings, it will be apparent to one of ordinary skill in the art that various alterations and modifications in form and details may be made in these embodiments without departing from the scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and equivalents to the claims are also within the scope of the following claims.

## Claims

1. A nicotine pouch comprising a nicotine pouch filler; and a packaging material, wherein a volume filling rate of the nicotine pouch filler is 60% to 75% of an internal volume of the nicotine pouch.

2. The nicotine pouch of claim 1, wherein the nicotine pouch has size with a vertical length of 11 mm to 17 mm and a horizontal length of 25 mm to 32 mm.

3. The nicotine pouch of claim 1, wherein the nicotine pouch has a weight of 0.3 g to 0.6 g.

4. The nicotine pouch of claim 1, wherein the internal volume of the nicotine pouch is 0.8 cm³ to 1.2 cm³.

5. The nicotine pouch of claim 1, wherein the packaging material is packaged in a lap-seal manner, and the packaging material is sealed with a width of 1 mm to 3 mm.

6. The nicotine pouch of claim 1, wherein the nicotine pouch has a thickness of 2.8 mm to 4.5 mm.

7. The nicotine pouch of claim 1, wherein the nicotine pouch filler comprises:
nicotine;
a release control excipient comprising at least one selected from a group consisting of cellulose and sugar alcohol;
a binder;
a pH adjuster; and
a flavoring agent.

8. The nicotine pouch of claim 1, wherein the nicotine pouch filler has a density of 0.34 g/cm³ to 0.74 g/cm³.

9. The nicotine pouch of claim 1, wherein the nicotine pouch filler has a content of 280 mg to 460 mg.

10. The nicotine pouch of claim 1, wherein the nicotine pouch filler has a filling density of 0.26 g/cc to 0.44 g/cc.

11. The nicotine pouch of claim 1, wherein the packaging material comprises a cellulose fiber; and a thermoplastic material fiber.

12. A method of packaging a nicotine pouch, the method comprising: wrapping a nicotine pouch filler with a packaging material; and sealing the packaging material,
wherein the nicotine pouch filler has a volume filling rate of 60% to 75% of an internal volume of the nicotine pouch.
